# EUROPEAN PATENT APPLICATION

(11) **EP 3 932 397 A1**
(43) Date of publication of application: **05.01.2022**
(21) Application number: 19917981.3
(22) Date of filing: 01.03.2019
(51) Int. Cl.: A61K 9/19, A61K 31/42, A61K 9/08, A61P 29/00

(54) **NON-STEROIDAL ANTI-INFLAMMATORY LYOPHILIZED PHARMACEUTICAL COMPOSITION**

(71) Applicant: Eurofarma Laboratórios S.A., 06696-000 Itapevi - SP (BR)
(72) Inventor: BILLI, Maurizio, CEP 04603-003 São Paulo - SP (BR); COSTA, Matheus Marques, CEP 06030-304 Osasco SP (BR); FOLCO, Emily Fernanda Freire, CEP 02420-000 São Paulo - SP (BR)
(74) Representative: KATZAROV S.A.
(86) International application number: PCT/BR2019/050067
(87) International publication number: WO 2020/176955

(57) **Abstract**

The present invention refers to parenterally administered pharmaceutical compositions comprising non-steroidal anti-inflammatory drugs or salts thereof, reconstitutable with specific reconstitution solution and free of buffering agent.

## Description

### Field of the Invention

The present invention refers to parenteral pharmaceutical compositions comprising non-steroidal anti-inflammatory drugs or salts thereof, reconstitutable with specific reconstitution solution and free of buffering agent.

### Background of the Invention

Nonsteroidal anti-inflammatory compounds (NSAIDs) are among the most used pharmacological agents in medical practice. They have a wide spectrum of therapeutic indications, such as: analgesia, anti-inflammation, antipyresis and prophylaxis against cardiovascular diseases.

Among the non-steroidal anti-inflammatory compounds, for example, the following can be cited: aceclofenac, ketorolac, ketoprofen, etodolac, diclofenac, naproxen, loxoprofen, nepafenac, nimesulide, ibuprofen, celecoxib, parecoxib, meloxicam, piroxicam, lornoxicam, tenenoxicam, among others.

For analgesia, NSAIDs are an important tool in the treatment of postoperative pain. When used alone, they have excellent analgesic efficacy for mild postoperative pain. In cases of moderate to severe pain, they can be used in association with other compounds, such as opioids, for example, reducing analgesic doses and the incidence of side effects of these compounds.

Acute pain is commonly associated with tissue damage caused by injury or surgery. Parenteral treatments are used in conditions of moderate to severe pain and when oral or rectal administration is not suitable.

In this case, the low aqueous solubility of most NSAIDs is a challenge to be worked on in the art (Brunton et al., Goodman and Gilman's The Pharmacological Basis of Therapeutics, 13th ed., 2009, p. 851). Another challenge to be faced is the stabilization of parenteral formulations, particularly avoiding the formation of particles (Tan et al., Quality-control analytical methods: particulate matter in injections: what it is and what are the concerns, International Journal of Pharmaceutical Compounding, vol. 10, no. 3, 2006).

Another challenge is the nucleophilic attack that the active principles may suffer from other compounds used in the pharmaceutical composition.

The Applicant verified that this is the case of active principles that have an amide group, which can undergo nucleophilic attack, characterized by a nucleophilic substitution, such as occurs in the compounds parecoxib, nepafenac, piroxicam, lornoxicam or tenoxicam, more particularly in the active ingredient parecoxib. The chemical name for parecoxib sodium is N-[[4-(5-methyl-3-phenyl-4-isoxazolyl)phenyl]sulfonyl]propanamide sodium salt, CAS number 198470-85-8, DCB number 06844, having molecular formula C₁₉H₁₇N₂O₄SNa, molecular weight 392.41 g/mol and the following formula I:

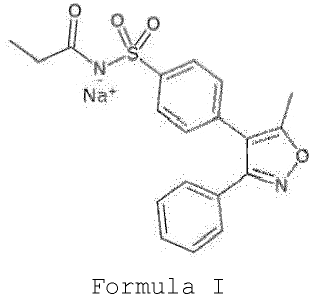

Parecoxib sodium is the prodrug of valdecoxib, that is, when administered, it is rapidly converted to valdecoxib.

Valdecoxib is also a member of the non-steroidal anti-inflammatory drug (NSAID) class, which, in animal models, has anti-inflammatory, analgesic and antipyretic activities.

The *in vivo* pharmacology of parecoxib is the same as valdecoxib. The mechanism of action of valdecoxib is believed to be the inhibition of prostaglandin synthesis mediated by the inhibition of cyclooxygenase 2 (COX-2). At therapeutic plasma concentrations in humans, valdecoxib does not inhibit cyclooxygenase 1 (COX-1). Due to the inhibition of central and peripheral COX-2, valdecoxib reduces the production of prostaglandins, important pain, and inflammation mediators.

Parecoxib is characterized by being a white or almost white powder, very soluble in water, slightly soluble in tetrahydrofuran and insoluble in methylene hydrochloride. The pH of 1% parecoxib in water at 25°C is of about 7.80 and the dissociation constant, pKa, 6.07. The asset is highly hygroscopic.

The solubility of the main degrading product, valdecoxib, is low. Therefore, a challenge in the development of products containing parecoxib is to obtain a stable formulation, in which the formation of valdecoxib is minimized.

In contrast, parecoxib sodium is highly soluble and its solubility is pH dependent, i.e., the solubility of the molecule increases with increasing pH. The solubility of parecoxib sodium at 20°C at pH 7.8 is of 18 mg/ml, and it is of 220 mg/ml at pH 8.3. At pHs above 7.5 the solubility is better than predicted according to the ionization of the molecule.

Additionally, parecoxib sodium is mostly found in neutral form at acidic pH values, but at pH values above 4.24 the predominant species is deprotonated. The deprotonated species is more soluble, that is, the solubility of parecoxib increases due to the increase in its deprotonated species.

According to the forced degradation study, the active is sensitive to oxidative, basic, and acidic conditions in relation to the formation of valdecoxib.

The formation of valdecoxib from parecoxib occurs by a reaction characterized by a nucleophilic attack. For the formation of valdecoxib from parecoxib to occur, a nucleophilic substitution reaction takes place, so the extent of this reaction depends on the strength of the nucleophile. In this case, the phosphate anion (PO₄³⁻) is a stronger nucleophile than water (neutral nucleophile).

Currently, products containing parecoxib in the form of its sodium salt are available in the form of lyophilized powder for injectable solution. For example, international application WO02080912 discloses a pharmaceutical composition in powder form comprising parecoxib sodium in a therapeutically effective total amount consisting of 30 to 90% by weight, a parenterally acceptable buffering agent in an amount of 10 to 60% by weight, and other parenterally acceptable excipient ingredients in a total amount of 0 to 10% by weight of the composition being reconstitutable in a parenterally acceptable liquid solvent to form an injectable solution.

It was verified that parecoxib, when in aqueous solution, and especially in the presence of certain excipients, has instability resulting in compounds with low solubility, such as valdecoxib.

The conversion of parecoxib to valdecoxib occurs over time and, because valdecoxib has low water solubility, the presence of particles resulting from its formation is undesirable in a solution for parenteral administration.

Thus, the residual moisture of the product in the form of lyophilic powder must be controlled and restricted to avoid the catalysis of the conversion described above by the presence of water.

Another important control related to residual moisture in the product is the amount of water intrinsic to the butyl cap that can be transferred to the product over its useful life.

Another characteristic of parecoxib is the conversion of parecoxib sodium into its acid form. Its formation is directly related to the pH of the finished product. Since this form has lower solubility when compared to parecoxib salt, this fact impacts the quality of the finished product and can cause pain and local irritation during administration. In some cases, particulate contamination can cause phlebitis, pulmonary embolism, pulmonary granuloma, immune system dysfunction, infarction and even death (Langille et al., Particulate Matter in Injectable Drug Products, PDA Journal of Pharmaceutical Science and Technology, 2013). In addition to the challenges presented above, in injectables, it is desirable to provide minimalist pharmaceutical formulations, that is, with as few excipients as possible. This is because the less excipient added to the formulation (for example, to control stability), the less patient exposure to excipients that can cause adverse reactions - even death -, as the supply of endotoxins is avoided (The United States Pharmacopeial Convention, Bacterial Endotoxins Test, p. 6.011-6.117, 2018).

However, this is a challenge that can hardly be overcome, since several supporting excipients, in general, is necessary to obtain adequate stabilization.

Thus, there is still a need in developing new minimalist pharmaceutical compositions that can carry NSAIDs, particularly parecoxib sodium, for parenteral application, in a stable and safe way over time, avoiding the formation of undesirable particles.

### Brief Description of the Figures

Figure 1 shows the graph of the content of the samples according to the present invention, in two batches EMI 078/18 and EMI 079/18, reconstituted with two reconstitution solutions soon after reconstitution and after 30 minutes and 24 hours of the reconstituted sample.
Figure 2 shows the degradation product (DP) graph of the samples according to the present invention, in two batches EMI 078/18 and EMI 079/18, reconstituted with two reconstitution solutions soon after reconstitution and after 30 minutes and 24 hours of the reconstituted sample.
Figure 3 shows the pH graph of samples stored at room temperature (15 to 30°C - control) and 30 days at 50°C/75% (exposed) according to the present invention, in two batches EMI 078/18 and EMI 079/ 18, reconstituted with two reconstitution solutions.
Figure 4 shows the graph of the content of the samples according to the present invention, in batch EMI 086/18, right after reconstitution with ringer lactate (T0) and after 24, 48 and 120 hours of the samples reconstituted and stored at room temperature (15 to 30°C) and in the refrigerator (2 to 8°C).
Figure 5 shows the degradation product graph of the samples according to the present invention right after reconstitution with ringer lactate (T0) and after 24, 48 and 120 hours of the samples reconstituted and stored at room temperature (15 to 30°C) and in refrigerator (2 to 8°C).
Figure 6 shows the degradation product graph of the reference drug, Bextra^{®}, right after reconstitution with three different reconstitution solutions (T0) and after 24 hours of the reconstituted samples stored at room temperature (15 to 30°C) and in a refrigerator (2 to 8°C).
Figure 7 shows the degradation product graph of the reference drug, Bextra^{®}, stored at room temperature (15 to 30°C - control) and 30 days at 50°C/75% (exposed).

### Description of the Invention

The present invention relates to a reconstitutable parenteral pharmaceutical composition comprising non-steroidal anti-inflammatory drugs, which consists of the active ingredient solubilized in an aqueous solvent and pH corrector.

Said composition is subjected to lyophilization, as well as subsequent reconstitution, with an appropriate agent.

Among the non-steroidal anti-inflammatory drugs according to the present invention, preferred are aceclofenac, ketorolac, ketoprofen, etodolac, diclofenac, naproxen, loxoprofen, nepafenac, nimesulide, ibuprofen, celecoxib, parecoxib, meloxicam, piroxicam, lornoxicam, tenoxicam, and/or its salts. In a preferred embodiment, the present invention contemplates those that can suffer nucleophilic attack characterized by a nucleophilic substitution, because they have the amide group, such as parecoxib, nepafenac, piroxicam, lornoxicam, tenoxicam or its salts, more preferably the active ingredient parecoxib or its salts, most preferably parecoxib sodium. Commercially available reconstitutable parenteral compositions generally comprise buffer in their formulations, for example, a phosphate buffer.

The Applicant verified that the presence of buffer, particularly phosphate in parecoxib formulations, a compound derived from the buffer commonly used in compositions available in the prior art, is harmful, either by causing leaching on the inner wall of the glass of the packaging container, or by catalyzing the formation of degradation products, in the particular case of parecoxib, for example, valdecoxib. This is observed for phosphate is a stronger nucleophile when compared to water.

However, the removal of the buffer, particularly the phosphate, causes instability in the formulation. Therefore, buffer and/or phosphate withdrawal is not an obvious solution to follow.

This is because, when removing the plug, which has a stabilization function, there will be no control over the degradation compounds that may be formed. Therefore, in addition to compromising stability, unknown compounds that are harmful to the patient's health may be formed.

In the particular case of parecoxib, the Applicant verified that the removal of the phosphate buffer provided the formation of an unexpected degradation compound, that is, parecoxib in its acidic form, making difficult the use of more trivial technical solutions for stabilization.

Thus, it was verified that the removal of buffer, particularly phosphate, from pharmaceutical compositions containing active principles susceptible to degradation causes precipitation of the active principle, forming degradation compounds, as is the case of parecoxib, which precipitates in its acid form of parecoxib, instead of forming valdecoxib.

A first obvious alternative to solve the precipitate problem would be to search for alternatives to solubilize the precipitate, either by mechanical means, promoting energy to solubilize, or by studying solvent versus solute.

However, unexpectedly, the Applicant verified that the formation of degradation compounds can be favored by the presence of the buffering agent itself used for stabilization. In the particular case of parecoxib, it was verified that valdecoxib in prior art formulations is favored by the presence of phosphates from the buffer used, a fact never considered in the prior art, even because this is the buffer of first choice, not being a trivial way to remove an agent that would have a known function and, until then, that would be efficient in stabilizing.

It was also verified that other excipients, normally present in commercial products, also influence the instability of parenteral compositions. Thus, providing minimalist parenteral formulations is highly desirable.

In this way, US patent 7695736, for example, teaches that the conversion of parecoxib to valdecoxib in a reconstitutable powder composition can be greatly reduced, reducing, or eliminating tablet formers, such as mannitol, from the composition. On this respect, the formation of valdecoxib was found in all the experiments using tablet formers at different concentrations and, on the contrary, the best result was obtained with the absence of tablet formers.

By logical reasoning, efficient injectable formulations to avoid the formation of degradation products could be those that contain active principle and water. As an example, it was observed that the composition that was more efficient in relation to the formation of valdecoxib under conditions of room temperature and heat was the one consisting of parecoxib and water for injectables, to solubilize the active ingredient. However, this presentation form would be technically unfeasible due to the formation of parecoxib in its acidic form.

This is because this acidic form of parecoxib has low solubility and is a white precipitate that is of easy visual detection, which would make the production of stable pharmaceutical compositions unfeasible.

In surprisingly efficient manner, it has been verified that the suitable pH for solubilization of parecoxib should be greater than 7. Thus, adjustment is necessary to provide an adequate pH that guarantees the stability of the product until the moment of use. Thus, to ensure total solubilization of the active ingredient and to ensure that the pH remains at the ideal level for product administration, a pH corrector was developed to adjust the initial solution in a pH range from 8.5 to 9.5.

In a particular embodiment the pH corrector is selected from strong bases, particularly sodium hydroxide, potassium hydroxide and/or calcium hydroxide, more particularly sodium hydroxide.

The composition according to the present invention is prepared by adding the active ingredient in part of the water and homogenized until total solubilization, followed by pH adjustment in a range of 8.5 - 9.5. The resulting solution is filled in a bottle with a cap previously dried and, subsequently, submitted to the lyophilization step.

In addition to the previously described advantages, it was also surprisingly verified that the freeze-drying cycle time was reduced by about 40%, allowing typical prior art cycles of around 36 hours to be reduced to about 25 hours, optimizing the productive process.

The composition of the present invention is suitable to be reconstituted with various solutions, for example, 0.9% sodium chloride solution, ringer lactate or other solutions with a pH above 4.5 and adequate osmolarity. Although it was previously expressly indicated as inappropriate in the literature, the reconstitution solution option that presents the best result according to the present invention, unexpectedly, is the lactate ringer solution, as it has the highest pH range (Crane et al., Stability of reconstituted parecoxib for injection with commonly used diluents, Journal of Clinical Pharmacy and Therapeutics 28, pp. 363-369, 2003, which suggests not using ringer lactate.).

Furthermore, in carrying out the present invention, it has been verified that the product in the form of lyophilized powder when reconstituted with the solutions according to the present invention is free of particles and suitable for use. On the other hand, glucose solutions are not recommended for reconstitution, as the product has particulate matter and parecoxib in its acidic form, due to the low pH.

It was observed that the prior art products do not recommend the use of a ringer lactate solution or a 5% glucose solution in ringer lactate for the reconstitution of the reference drug, as precipitation will occur. Crystal formation is observed within 54 to 72 hours, of calcium phosphate nature, a crystal of low solubility. Phosphate comes from prior art buffering agent, sodium phosphate, and calcium is present in the composition of ringer lactate in the form of salt, calcium chloride.

Thus, the present invention relates to a minimalist pharmaceutical composition comprising at least one non-steroidal anti-inflammatory drug (NSAID), particularly selective cyclooxygenase-2 (COX-2) inhibitors, such as those selected from celecoxib, rofecoxib, etoricoxib, lumiracoxib and/or parecoxib, or salts thereof. In a preferred embodiment the present invention comprises parecoxib, particularly in the form of parecoxib sodium, solubilized in an aqueous solvent and pH corrector to adjust the pH of the solution.

In a preferred embodiment, the present invention contemplates a parenteral pharmaceutical composition consisting of parecoxib sodium, water for injections and pH corrector sodium hydroxide, lyophilized and reconstitutable with ringer lactate solution.

In an even more preferred embodiment, the present invention contemplates about 15 to 25 mg of parecoxib sodium and sufficient amount of sodium hydroxide to obtain a pH between 8.5 and 9.5 in about 1 ml of water, for a volume of fill about 2 ml.

Furthermore, water has been verified to be a catalyst in the formation of parecoxib in acidic form. Thus, the composition according to the present invention was developed so that the product after freeze-drying has a smaller amount of residual water and a smaller amount of water coming from its primary packaging.

It was verified that a smaller amount of water guarantees the stability of the product in relation to the formation of acidic parecoxib. Therefore, the amount of adequate residual moisture is less than 1.0%.

In this same sense, it was also observed that the presence of water in the butylic cap used in the packaging of this type of product also contributes negatively to the problems mentioned above.

The primary packaging consists of an ampoule bottle and a butylic cap. The butylic cap has a certain amount of intrinsic water that can be transferred to the lyophilic powder. Therefore, the present invention contemplates the use of optionally dry and uncoated butylic caps, which have been subjected to a drying process to reduce the amount of water present in them.

Thus, the reconstitutable parenteral pharmaceutical composition of parecoxib sodium according to the present invention, free of buffering agent, in the form of lyophilic powder, does not show formation of valdecoxib above the technically acceptable limit (0.2%). In addition, there is no formation of parecoxib in the acidic form when reconstituted, since the reconstitution solution promotes an adequate pH to obtain a clear solution, free of precipitate.

After reconstitution stable products are obtained for intravenous and intramuscular administration.

The composition according to the present invention is prepared from the solubilization of the active principle in a therapeutically effective amount according to the prior art, in water for injectables, followed by pH adjustment and lyophilization, through processes that are commonly applicable in the pharmaceutical industry and known by the person skilled in the art.

The following examples serve to illustrate aspects of the present invention without, however, having any limiting character. Tests were carried out with the active ingredient parecoxib sodium only as an example.

### Examples

### Example 1 - Preparation of the compositions according to the present invention.

**Table 1. Composition according to the present invention.**

| Ingredient | mg/ml | pH |
|---|---|---|
| Parecoxib sodium | 21.18 | 8.5 - 9.5 |
| Water for injectables | 0.80 | |
| Sodium hidroxide | q.s. | |
| Water for injectables q.s.p. | 1.00 | |

The active ingredient was added in part of the water and homogenized until complete solubilization. The pH was checked, and the pH adjustment was carried out with sodium hydroxide, considering that enough sodium hydroxide should be used to adjust the pH in a range of 8.5 - 9.5. The final volume was adjusted with water considering the final batch size. The product was filled in a colorless glass bottle. The bottles were semi-corked with an uncoated butylic cap, subjected to prior drying, and they were placed in the lyophilizer for the lyophilization step. The product was lyophilized in order to obtain the lowest possible residual moisture.

### Example 2 - Comparative test of typical freeze-drying cycle times (freeze-drying curve).

The comparative test was based on data from patent document US 7695736, table 3, where it is observed that the typical lyophilization cycle is of 36 hours.

**Table 2. Composition lyophilization cycle as per Example 1.**

| Phase | Description |
|---|---|
| Freezing | Preparation: 0°C in 1 min, 0°C to - 45°C in 1 h kept at -45°C for 3 h |
| Primary drying | -45°C to 25°C in 2.5h kept at 25°C for 14.5h 350 µbar vacuum |
| Secondary drying | 25°C to 50°C in 1h kept at 50°C for 3 h 350 µbar vacuum |
| Total cycle time | 25 hours |

### Example 3 - Stability of the composition according to the present invention at high temperature and room temperature.

Promising prototypes were incubated at 50°C/75% for 30 days and analyzed as regards their content, degradation product and pH.

The tested compositions are described below and were prepared according to Example 1.

**Table 3. Formulations used in the study.**

| Composition 1 | | |
|---|---|---|
| Material | mg/ml | pH |
| Parecoxib sodium | 21.18 | 8.20 |
| Water for injectables | 0.80 | |
| Water for injectables q.s.p. | 1.00 | |

| Composition 2 | | |
|---|---|---|
| Material | mg/ml | pH |
| Parecoxib sodium | 21.18 | 9.64 |
| Water for injectables | 0.80 | |
| Sodium hidroxide | q.s. | |
| Water for injectables q.s.p. | 1.00 | |

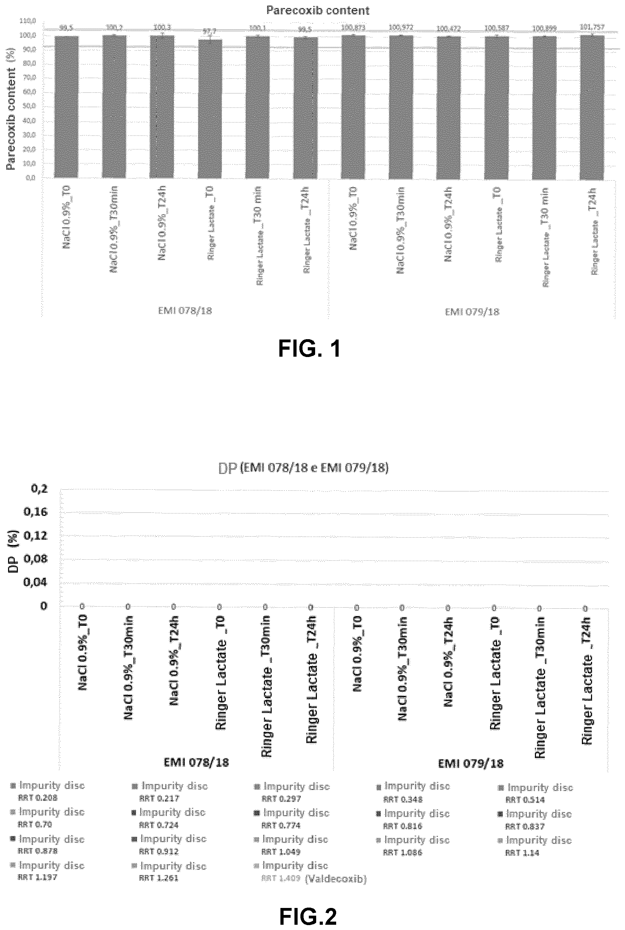

Figure 1 shows the graph of the content of the samples according to the present invention, in two batches EMI 078/18 and EMI 079/18, reconstituted with two reconstitution solutions soon after reconstitution and after 30 minutes and 24 hours of the reconstituted sample.

Figure 2 shows the graph of degradation product of samples according to the present invention, in two batches EMI 078/18 and EMI 079/18, reconstituted with two reconstitution solutions soon after reconstitution and after 30 min and 24 hours from reconstituted sample.

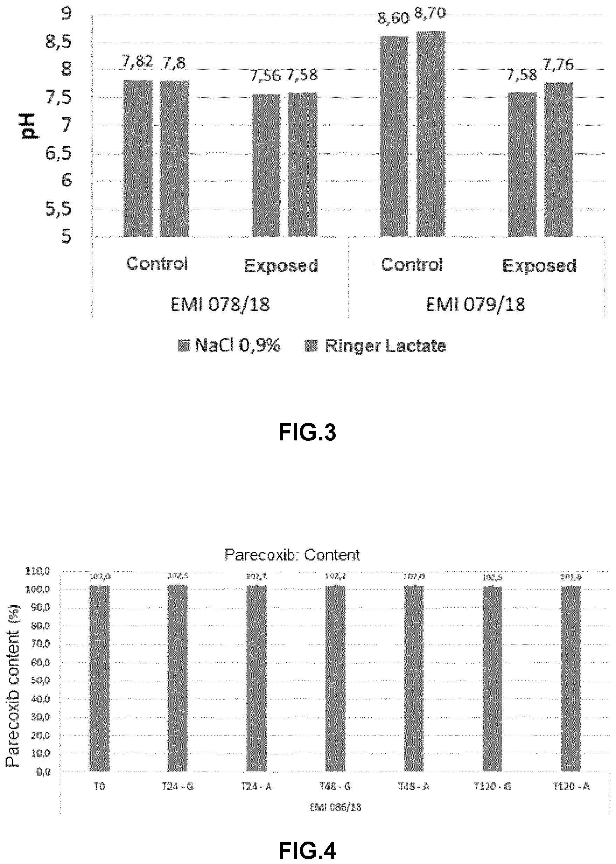

Figure 3 shows the pH graph of samples stored at room temperature (15 to 30°C - control) and 30 days at 50°C/75% (exposed) according to the present invention, in two batches EMI 078/18 and EMI 079/18, reconstituted with two reconstitution solutions.

### Example 4 - Stability of the composition according to the present invention at low temperature and room temperature

The proposed formulation was reconstituted and studied for five days, at room temperature and in a refrigerator, and it was stable.

The commercial product Bextra^{®}, by Pfizer, was used as a comparative control. The product's commercial leaflet provides information that the product should not be stored for more than 24 hours after reconstitution and should not be refrigerated or frozen.

The proposed product was submitted to a time and temperature not recommended by the manufacturer of the reference drug and no change in the quality of the product was observed.

The stability of the prior art product was tested under the following conditions:
TO: after reconstitution;
T24: 24 hours after reconstitution;
Amb.: room temperature;
Refrig.: temperature of 2 - 8°C;
Control: stored at room temperature;
Exposed: stored 30 days at 50°C.

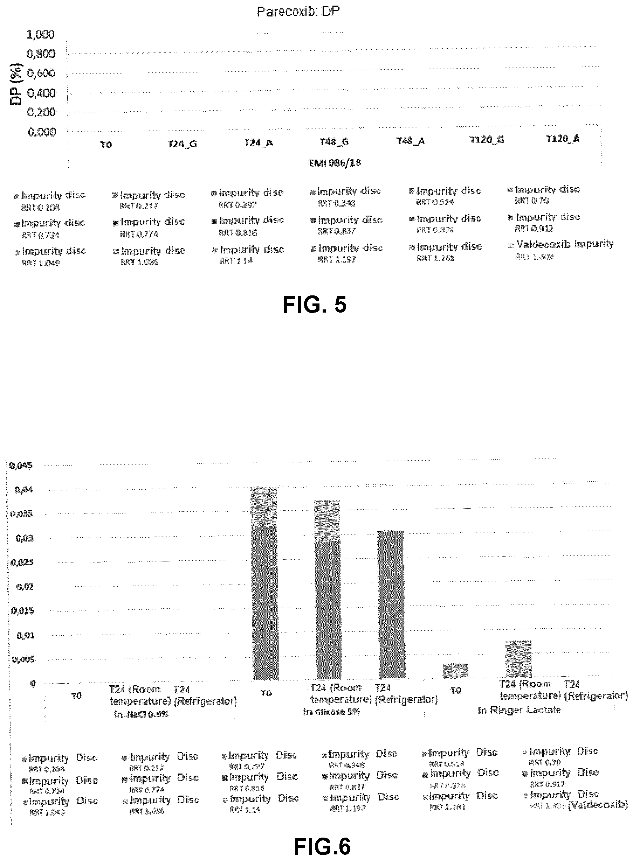

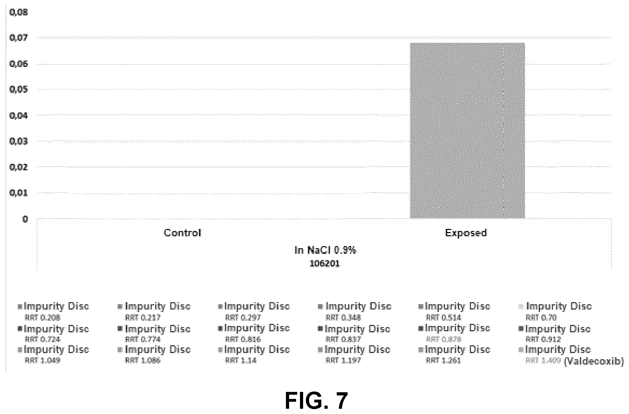

The results are shown in Figures 6 and 7.

Comparative assays were carried out with the composition of the present invention, which was prepared according to the procedure described in Example 1:

| Ingredient | mg/ml |
|---|---|
| Parecoxib sodium | 21.18 |
| Water for injectables | 0.80 |
| Sodium hidroxide | q.s. |
| Water for injectables q.s.p. | 1.00 |

Where:
T0 = zero time;
T24 - G = time of 24 hours in refrigerator;
T24 - A = time of 24 hours at room temperature;
T48 - G = time of 48 hours in refrigerator;
T48 - A = time of 48 hours at room temperature;
T120 - G = time of 120 hours in refrigerator;
T120 - A = time of 120 hours at room temperature.

Figure 4 shows that the content was approximately 102% at all times. In no time there was formation of valdecoxib.

Figure 5 shows the degradation product graph of the samples according to the present invention right after reconstitution with ringer lactate (T0) and after 24, 48 and 120 hours of the samples reconstituted and stored at room temperature (15 to 30°C) and in refrigerator (2 to 8°C), where A stands for environment and G stands for refrigerator.

The test results demonstrate that the formulation according to the present invention can be used in up to 5 days without losing its initial characteristics, allowing greater flexibility to the user if the product is reconstituted and not used immediately. In addition, the minimalist formulation according to the present invention allows for greater chemical and microbiological control of the excipients used. This assignment is advantageous for injectable products.

It should be understood that the embodiments described above are merely illustrative and that any modification thereon may occur to a person skilled in the art. Accordingly, the present invention shall not to be considered limited to the embodiments described in this specification.

## Claims

1. Lyophilized non-steroidal anti-inflammatory pharmaceutical composition **characterized by** the fact that it comprises at least one non-steroidal anti-inflammatory and pH correcting agent.

2. Parenteral non-steroidal anti-inflammatory pharmaceutical composition **characterized by** the fact that it consists of at least one non-steroidal anti-inflammatory agent solubilized in an aqueous solvent and pH corrector.

3. Pharmaceutical composition, according to any of claims 1 or 2, **characterized in that** the non-steroidal anti-inflammatory agent is selected from aceclofenac, ketorolac, ketoprofen, etodolac, diclofenac, naproxen, loxoprofen, nepafenac, nimesulide, ibuprofen, celecoxib, meloxicam, piroxicam, lornoxicam, tenoxicam, parecoxib and/or salts thereof.

4. Pharmaceutical composition, according to claim 3, **characterized in that** the non-steroidal anti-inflammatory agent is selected from parecoxib, nepafenac, piroxicam, lornoxicam, tenoxicam and/or salts thereof.

5. Pharmaceutical composition, according to claim 4, **characterized in that** the non-steroidal anti-inflammatory agent is parecoxib.

6. Pharmaceutical composition, according to claims 5, **characterized by** the fact that parecoxib is in sodium form.

7. Pharmaceutical composition, according to any of claims 1 or 2, **characterized in that** the pH corrector is selected from at least one strong base.

8. Pharmaceutical composition, according to claim 7, **characterized in that** the strong base is selected from sodium hydroxide, potassium hydroxide and/or calcium hydroxide.

9. Pharmaceutical composition, according to claim 8, **characterized in that** the strong base is sodium hydroxide.

10. Pharmaceutical composition, according to any of claims 1 to 9, **characterized in that** the pH of the composition is in a pH range from 8.5 to 9.5.

11. Reconstitutable parenteral pharmaceutical composition of non-steroidal anti-inflammatory, **characterized in that** it is obtained from the lyophilization of the pharmaceutical composition as defined in one of claims 2 to 10.

12. Pharmaceutical composition, according to any of claims 1 or 11, **characterized in that** it is reconstituted with ringer lactate and/or 0.9% sodium chloride.

13. Pharmaceutical composition, according to any of claims 1 or 11, **characterized in that** the amount of residual water in the composition is less than 1%.

14. Pharmaceutical composition, according to any of claims 2 to 10, **characterized in that** it consists of about 15 to 25 mg of parecoxib sodium and a sufficient amount of sodium hydroxide to obtain a pH between 8.5 and 9.5 in about 1 ml of water, for a filling volume of about 2 ml.

15. Stable pharmaceutical product **characterized in that** it comprises a pharmaceutical composition as defined in one of claims 2 to 10 packaged in a vial and dry butylic cap.
